# EUROPEAN PATENT SPECIFICATION

(11) **EP 1 727 909 B1**
(45) Date of publication and mention of the grant of the patent: **10.12.2008**
(21) Application number: 05730305.9
(22) Date of filing: 21.03.2005
(51) Int. Cl.: C12Q 1/44, C12Q 1/04, C12Q 1/18

(54) **METHOD AND TEST-KIT FOR THE DETECTION AND QUANTIFICATION OF ORGANISMS**
VERFAHREN UND TESTKIT ZUM NACHWEIS UND ZUR QUANTIFIZIERUNG VON ORGANISMEN
PROCEDE ET KIT DE TEST DESTINES A LA DETECTION ET LA QUANTIFICATION D'ORGANISMES

(30) Priority: 22.03.2004 EP 04006833
(43) Date of publication of application: 06.12.2006
(73) Proprietor: Evonik Goldschmidt GmbH, 45127 Essen (DE); Proteus S.A., 30000 Nîmes (FR)
(72) Inventor: ALLEF, Petra, 53121 Bonn (DE); GRÜNING, Burghard, 45134 Essen (DE); RAVOT, Gilles, F-30900 Nimes (FR); WAHLER, Denis, F-30132 Caissargues (FR)
(74) Representative: Bredema
(86) International application number: PCT/EP2005/002975
(87) International publication number: WO 2005/093089

(56) References cited:
- DE-A1- 19 608 320
- US-A- 4 603 108
- US-A- 5 098 830
- US-A1- 2002 031 795
- US-A1- 2003 199 017
- US-A1- 2004 048 326
- LANGLET S ET AL: "A new gel tube method for the direct detection, identification and susceptibility testing of bacteria in clinical samples" FEMS MICROBIOLOGY LETTERS, vol. 170, no. 1, 1 January 1999 (1999-01-01), pages 229-235, XP002343236 ISSN: 0378-1097
- WEI G X ET AL: "Proteolysis and utilization of albumin by enrichment cultures of subgingival microbiota" ORAL MICROBIOLOGY AND IMMUNOLOGY, vol. 14, no. 6, December 1999 (1999-12), pages 348-351, XP002343237 ISSN: 0902-0055
- LAGARDE D ET AL: "High-throughput screening of thermostable esterases for industrial bioconversions" ORGANIC PROCESS RESEARCH AND DEVELOPMENT, CAMBRIDGE, GB, vol. 6, no. 4, 27 June 2002 (2002-06-27), pages 441-445, XP002288681
- DATABASE WPI Section Ch, Week 200308 Derwent Publications Ltd., London, GB; Class B05, AN 2003-084866 XP002288684 & JP 2002 326942 A (NONOGAWA SHOJI KK) 15 November 2002 (2002-11-15)
- JAEGER K-E ET AL: "BACTERIAL LIPASES" FEMS MICROBIOLOGY REVIEWS, ELSEVIER, AMSTERDAM, NL, vol. 15, no. 1, January 1994 (1994-01), pages 29-63, XP000490500 ISSN: 0168-6445
- GROGNUX J ET AL: "Universal chromogenic substrates for lipases and esterases" TETRAHEDRON: ASYMMETRY, ELSEVIER SCIENCE PUBLISHERS, AMSTERDAM, NL, vol. 15, no. 18, 20 September 2004 (2004-09-20), pages 2981-2989, XP004575083 ISSN: 0957-4166

## Description

### Field of the invention:

The invention relates to a novel method for the detection and/or quantification of germs responsible for axillary odour, cultivable or not cultivable, by the detection of a lipase activity shared by said germs. The invention also relates the application of said method in the cosmetic field. More specifically the invention is concerned with methods for the detection and/or quantification of said germs, cultivable or not cultivable, collected directly from a bodily surface, namely skin surface, preferably armpits, without previously culturing the microorganisms. Moreover, the invention relates to methods for the evaluation of the effect of a substance of interest expected to act on said germs, cultivable or not cultivable.

### Background of the invention:

Detection and/or quantification of organisms presence, particularly microbial presence, is very important in various domains.

Many organisms, such as microorganisms, flourish under many conditions and colonize many kinds of environments, such as surfaces of objects, host tissues, food or liquids.

Such colonization can cause problems or, in contrary, have beneficial effects, depending on the type of organisms.

For example, problems can arise from the development and dissemination on or into host tissues causing many diseases of humans, animals and plants. Another example is the development of bacteria in food, such as *Listeria monocytogenes,* which contributes to food spoilage and to the risk of food poisoning. Specific enzymes of microorganisms, e.g. lipases or esterases on surfaces such as human skin or hair, are responsible for odour development, the development of acne or the generation of dandruff on the scalp.

Concerning the beneficial effects of development of organisms, and more specifically microorganisms, they can be useful as additives in certain cases, such as, for example, in the improvement of biological fertilizer properties, for destruction of pollution, as food additives, such as in cheese or grapes to produce wine. In this last case, for example, specific wines need development of specific fungi on grapes that will start the rot and give a particular taste to the wine. The detection and quantification of said fungi on grapes can be helpful to choose the collect day of grapes.

In addition to microbial examination of samples, collected into liquids or on surfaces, it is often necessary to rapidly characterize the microbial content of such samples in order to ascertain appropriate treatment: said treatment can be for decontamination or to increase, on the contrary, the growth of microorganisms.

Classically, organisms, more specifically microorganisms, which are searched in samples are detected and quantified by culturing on solid growth media until colonies are visible, or by sterility testing in liquid media. Said cultures on growth media are typically used to detect their presence, to test their enzymatic activities, and the inhibitory or activator effect of a specific substance on their growth. Such methods are only suitable for the detection or quantification of cultivable organisms.

In addition, detection and/or quantification of the presence of a group of organisms, particularly of a group of microorganisms, cultivable or not cultivable relies on time-consuming biological tests.

US-A-5,789,191 describes a cosmetic or dermatological method for detecting and/or selectively quantifying individual microorganisms, and/or whole groups of microorganisms, which are present on human or animal skin. However, this method comprises a step of addition of a sample treated with a deinhibiting medium, to a culture medium which exhibits favorable growth conditions for a defined group of microorganisms but unfavorable growth condition for other microorganisms. Thus, this method is specific, time-consuming and laborious because of the necessity of a culture step that must last long enough for the microorganisms to multiply and to produce metabolic products; these metabolic products are collected and measured.

The process of previous culture may take days to weeks for the most common bacteria, such as, for example *Mycobacterium tuberculosis,* and even up to one year for *Mycobacterium leprae.* Furthermore, there are bacteria for which it is up to now impossible to cultivate *in vitro* such as, for example *Helicobacter heilmannii.* Thus, some viable but poorly-cultivable or non-cultivable microorganisms may never grow *in vitro* due to particular, and as yet undetermined, growth requirements.

Some viable microorganisms are highly virulent and pathogenic, thus causing important public health problems, notably toxic infection of digestive tract, such as the strain *Vibrio cholerae, Listeria monocytogenes.*

Some others are known to be highly pathogenic, e.g. *Bacillus anthracis, Yersinia pestis, Clostridium Botulinum, Brucella sp. , Burkholderia mallei, Francisella tularensis, Clostridium perfringens*, and being cultivable in very particular conditions of safety. The rapidity of the microbial detection of such pathogens is thus vital for the population and for the soldiers, their equipment, instruments, clothes, skin and hair.

Moreover, some microorganisms frequently remain viable, and thus potentially pathogenic, even after a treatment (e.g. with antimicrobial substances for deodorants, sterilization treatment in hospital rooms, ambulance, for apparatus, surgical instruments, etc.), yet are sufficiently weakened so that detection by conventional assay protocols may require a non-selective recovery step (pre-enrichment) followed by a selective enrichment step to allow growth of the targeted microorganisms while growth of competing microorganisms is inhibited. Such additional steps can significantly increase the time required to perform the assay.

Other assays based on immunoassays, or on PCR can be used for detecting organisms, and more specifically microorganisms. However, these methods give no information about viability of the detected organisms since viable and non viable organisms are equally detected.

Microorganisms presence can be assayed using the RT-PCR method, by indirect detection of the mRNA encoding an enzymatic activity. In this procedure an RNA sample is copied into cDNA, which is then amplified by PCR. The amplified DNA fragments can be analyzed e.g. by agarose gel electrophoresis and the amount of produced fragments is essentially linked to the amount of target RNA and in certain cases to the amount of protein. This method, however, is costly, time consuming and the quantification is often not precise. Moreover, the RT-PCR is only an indirect measurement of the expression level of a targeted enzymatic activity. Additionally, this method does not give any information as to whether an enzyme is present in an active form or not.

The APIZYM system provides a method for microorganisms detection through their enzyme activities (EP-A-1 451 775). However, the system sold by BioMérieux is based on a plastic strip holding a number of miniature biochemical test tubes including fermentation and enzymatic tests (usually about twenty tests). The tubes containing enzymes substrates are inoculated with a suspension of culture. The reactions either produce directly a color change or are revealed by the addition of reagents. Results are treated and interpreted either by comparing the qualitative profile list included in the instruction manual or by computer analysis using APILAB software. This system requires a culturing step, and is therefore time-consuming, laborious and costly.

The ATP bioluminescence assay allows for a sensitive detection and quantification of viable microorganisms. Microorganism levels are measured through the amount of ATP they contain. It was found that the firefly produces its characteristic glow by utilizing ATP in a reaction with the luciferase enzyme and luciferin substrate to release energy as light. The light produced by the reagents can then be quantified. However, this method is not specific since ATP is a universal energy molecule common to all living cells and organisms and is therefore present not only in microorganisms but also on many residues and surfaces such as the skin.

WO-A-2004/015141 describes a method to assess the presence and viability of microorganisms, by administering an antimicrobial agent to a population of microorganisms having a first and second marker, quantifying a first and a second marker, and determining the ratio between the quantity of the first and second marker. Said markers of the bacterial organism can be chromosomal or plasmid DNA, mRNA or ribosomal RNA. A concordant result indicates the presence of viable microorganisms, whereas a discordant result indicates the presence of non-viable microorganisms. Following administration of the antimicrobial to the microorganisms having a first and second marker, the markers are quantified via real-time quantitative PCR (Polymerase Chain Reaction). However, this method is technically time-consuming, laborious and costly, because of the cost of quantitative RT-PCR reagents and equipment.

Other methods are using the detection of DNA or RNA of the targeted organisms, and more specifically of the targeted microorganisms.

WO-A-2004/009843 describes a kit for detecting microorganisms, containing at least one oligonucleotide for at least one species or a group of species of microorganisms which occurs on the skin and a method in which the inventive kit is used. However, this method is laborious because of the determination step of each oligonucleotide, and consists in *in situ* hybridization that is time-consuming, costly and yields false positive and false negative results. Moreover, methods using oligonucleotides hybridization are not suitable in basic and highly saline media.

WO-A-00/75636 describes a method enabling high-throughput screening of cells for taxonomic classification, without cultivation step. However, this method which is a whole-cell *in situ* method, requires complex instrumentation, thus is very costly, and needs the creation of probe sets labeled with fluorophores for simultaneous hybridization against 16S or 18S rRNA and other targets. Moreover, sequences of DNA/RNA of organisms must be known before the analysis. Description of this document precises notably that cells need not be viable.

WO-A-02/29096 describes a method for detecting organisms in a collected sample but said method is based on the detection of DNA from organisms of said sample, consisting notably in preparing one or several probes specific of said organisms, and extracting DNA of said organisms contained in the sample, even if no previous culture of organisms present in the sample is described.

In any DNA or RNA detection based assay described above, the sequences of DNA/RNA of the targeted organisms must be known before the analysis. Moreover, it may be difficult to detect a group of organisms, as they should share a common DNA or RNA sequence.

On the other hand, other assays based on the use of transformable substrates have been described for the detection of microorganisms.

WO-A-01/92563 generally describes a method for detecting and quantifying a chemical transformation and more specifically an enzymatic transformation in a sample. The method uses a stable substrate, which is enzymatically transformed and quantified. However, this method is not used for the detection and quantification of the expression of a specific enzyme or the detection or quantification of microorganisms expressing said enzyme, and which are located on an ecosystem or a surface, e.g. the skin.

JP 2002 326942 discloses a preparation for external use for skin containing antimicrobial compound useful in the treatment of acne and rough skin. The inhibition rate of said preparation (test-substance solution) is evaluated using *Pseudomonas* microbe derived lipase and 4-methyl umbelliferyl oleate. The lipase inhibitory action of said preparation is tested with only a lipase reference from a single microbe. Therefore it does not reflect the real efficiency of the preparation as the test is not carried out with a real sample collected from the skin but with a reference microbe from *Pseudomonas.*

Efficiency testing of raw materials for personal care products and cosmetic formulations are a main issue in the industry. The efficiency testing of deodorants for example is generally evaluated by three approaches: *in vitro* microbiological evaluation, chemical analysis and sensory evaluation of the odour. However, in case of evaluation of substances affecting axillary malodour, the chemical analysis method is limited by the complex and variable nature of said malodour. Only single chemicals can be detected, which are known to be part of the axillary malodour.

In order to avoid the development of axillary malodour or dandruff, deodorants and anti-dandruff shampoos often contain anti-microbial substances. These inhibit the growth of bacteria and thereby reduce the amount of enzymes responsible for the development of axillary malodour or the formation of dandruff.

EP-B-0 666 732 describes new antimicrobial substances for deodorants and *in vitro* tests for their efficiency. The antimicrobial activity against single germs like *Corynebacterium xerosis* is investigated. This test is time consuming, since it takes up to 48 h. Moreover, the test requires that germs be cultivated first and is therefore expensive. Additionally, the results are sometimes falsely negative.

EP-B-1 250 842 describes new antimicrobial substances for deodorants and their efficiency testing *in vivo* by sensory evaluation. Sniff tests, however, are cost intensive, because specially trained experts are needed. Additionally, the quantification of these results can be difficult and inprecise.

A new approach to the assessment of deodorants deals with the counting of the axillary germ population by cultivation of said germs with and without treatment of the armpits. This method is efficient, but also time consuming, because the incubation of the germs takes 2 to 4 days (A. R. Cox, J. Soc. Cosmet. Chem. 38, 223-231 (1987)).

The application of certain antimicrobial substances to the skin is directly linked to the reduction of lipase activity. Hence, the efficiency of those antimicrobial agents and/or deodorant formulations correlates with the reduction of lipase activity, or more precisely, with the effect on the viability of microorganisms expressing said lipases.

Furthermore, anti-dandruff formulations are usually evaluated by determination of the antimicrobial activity against *Malassezia furfur* or by clinical trials. Both methods are time consuming and expensive.

### Disclosure of the invention:

In the light of the foregoing, wherein the methods for determination and/or quantification of a group of organisms, specifically a group of microorganisms, cultivable and not cultivable, are generally tedious, costly and time consuming and sometimes impossible. It would be desirable to provide methods for detecting and quantifying a group of organisms, or microorganisms, that do not suffer from the above mentioned drawbacks, notably that do not necessitate a previous culture step.

To solve this technical problem, the invention discloses a method consisting of collecting a sample obtained from a bodily surface, namely skin, and then detecting or quantifying the presence of germs responsible for axillary odour, cultivable or not cultivable in this sample through a specific lipase activity shared by said germs. The detection is made without any previous culture of said sample and with very simple and cheap equipment. This detection and/or quantification could be considered as a pre-screening assay to detect said germs sharing the same lipase activity among other groups of organisms which do not have said lipase activity.

Thus, the present invention provides an *in vitro* method for the evaluation of the efficiency of a substance of interest in cosmetic formulations comprising:
a) providing a sample obtained from a bodily surface to which a cosmetic composition comprising a substance of interest has been applied, wherein the bodily surface is skin;
b) evaluating the presence of germs responsible for axillary odour sharing lipase activity by contacting said sample with a lipase substrate, and detecting and quantifying the amount of transformed substrate, wherein said sample is not submitted to a culture step prior to said contacting;
c) evaluating said efficiency by comparing with results without previous application of said composition.

More preferably the lipase substrate is a C3 to C18 ester, preferably a 2-hydroxy-4-p-nitrophenoxy-butyl carboxylic acid ester, and particularly preferred it is 2-hydroxy-4-p-nitrophenoxy-butyl hexanoate or 2-hydroxy-4-p-nitrophenoxy-butyl decanoate.

### Definitions:

In the following, several terms used throughout the description will be explained:

### Organisms:

The term "organisms" means any organisms or microorganism, such as Bacteria, yeast, fungi, viruses, protists (protozoan, micro-algae), archaebacteria, and eukaryotes. Said organisms means parasites too. Said organisms are considered to be "viable", even if said organisms are technically not cultivable, as long as they are capable of colonization, infection, replication, and propagation in the presence of a suitable environment.

### Group of organisms:

The term "group of organisms" means organisms cultivable or not cultivable, sharing a common enzymatic activity. In the field of the invention, this term means germs responsible for axillary odour, sharing a lipase activity.

### Not cultivable organisms:

Organisms that are up to now impossible to cultivate *in vitro* with actually known techniques.

Moreover, it also concerns organisms, more specifically bacteria, that have lost the capacity to produce a colony on a nutritive solid medium, but does not necessarily mean that these organisms are dead. Therefore, such organisms are viable (intact and metabolically active cells) but not cultivable because they are unable of reproduction (taking into consideration sexual reproduction or not) in known laboratories growth conditions.

### Enzymatic activity:

Any catalytic ability of an enzyme that is of interest to act on substances used in the field of chemistry, biology, pharmaceutics, veterinary, cosmetics, food industry, industrial and institutional cleanliness, military and related fields. Preferred examples for such enzymes are oxidoreductases, transferases, hydrolases, lyases, isomerases, ligases, or any combination thereof. In specific embodiments, such enzymatic activities can be proteases, esterases, or lipases or any combination thereof. In the field of the invention, such enzymatic activity is lipase.

### Substance of interest:

Any substance, composition of substances, extract of substance, or formulation that may have an effect on a targeted group of organisms. The substance, composition of substances, extract of substance, or formulation can also acquire an activity after reacting with said group of organisms. The substance can be, for example, an activator or an inhibitor of growth or viability of organisms. In the field of the invention, a substance of interest may have an effect on germs responsible for axillary odour

### Transformable substrate:

Specific substrate transformed by the enzymatic activity shared by a group of organisms which is searched for. In the field of the invention, such transformable substrate is a lipase substrate transformed by the lipase activity shared by germs responsible for axillary odour

### Sampling tool:

Sampling tools used within the meaning of the present invention are all those generally known by persons skilled in the art, specifically those which can rinse off and collect fluid, which suck off fluid and/or solid matter, like swabs, syringes, pipettes, smear tests or tape strippings.

### Direct detection/quantification:

As used herein, this term means that the collected group of organisms are not cultured *in vitro* before the sample (that is suspected of containing said group of organisms sharing the same enzymatic activity) is subjected to the determination of the enzymatic activity.

### Detailed description of the invention:

The present invention provides a simple method for a specific, sensitive, fast and cheap detection and/or quantification of the presence of' germs responsible for axillary odour, cultivable or not cultivable, in a sample obtained from a bodily surface, namely a skin surface, preferably armpits, through the measurement of the shared lipase activity.

Moreover, the object of the method of the invention is that no previous culture of the collected sample is necessary in any step of the method of the invention, thus the man skilled in the art does not need to know, search, develop or use growth media of said germs (no preparation of culture media, no adaptation of the growth conditions, etc.) allowing detection and/or quantification of said germs that are poorly cultivable or not cultivable.

It is well known that some groups of organisms, specifically some groups of microorganisms are known in prior art as sharing a particular enzymatic activity. The choice of said enzymatic activity is realized regarding of organisms which could be known to be mainly present on surfaces or liquids, as described hereinafter.

To determine the shared enzymatic activity of a group of organisms, the man skilled in the art analyses the known bibliographic, genomic, phylogenetic, proteomic, physiological data in order to define which enzymatic activity may be shared by a group of organisms. In some specific applications, the man of the art can also use the available informations on the ecosystem ecology in/on which organisms are suspected to be present. As an example, for skin surfaces, the man of the art knows that skin surface contains lipids that are used by natural skin micro-organisms as energy sources for their growth, which means that these micro-organisms contain lipases or esterases as a shared enzymatic activity.

Each enzymatic activity is characterized by its catalytic ability to transform a specific substrate, or several specific substrates.

A step of the method is to select a transformable substrate, corresponding to the lipase activity shared by germs responsible for axillary odour, cultivable or not cultivable, which is searched.

The step of selection of the transformable substrate corresponding to said lipase activity is realized using sources like chemicals catalogues (such as Fluka, Sigma, Acros, Merck, etc.) or bibliography, or even by designing new specific substrates.

In specific embodiments of the present invention, the use of a single transformable substrate or of a cocktail of transforable substrates can be considered. Said transformable substrate(s) must be as sensitive and specific for the shared lipase activity as possible. Such specific and highly sensitive and stable transformable substrates, including derivatives of 4-nitrophenyloxy-1,2-butanediol or 4-umbelliferyloxy-1,2-butanediol, have already been described in PCT application WO-A-0192563.

As an other example, Ferencko. et al., 2004 indicates that the diacetyl fluorescein is a fluorogenic substrate which is hydrolyzed nonspecifically by esterases, lipases and proteases. The use of this substrate in the method of the invention can then be useful to detect germs responsible for axillary odour, cultivable or not cultivable, sharing lipase activity among other groups of organisms that do not express such lipase activity.

Long-chain fatty acid esters of 7-(3,4-dihydroxybutyloxy)-2H-1-benzopyran-2-one are also described to be exceptionally sensitive and selective fluorogenic substrates for lipases and esterases (Nyfeler et al., 2003). Therefore, said substrates can be used in specific embodiments of the method of the present invention, to specifically detect germs responsible for axillary odour sharing lipase activity.

Moreover, long-chain fatty acid esters of 7-(3,4-dihydroxybutyloxy)-2H-1-benzopyran-2-one fluorogenic substrates are described as being at least by one order of magnitude more sensitive to lipases than the commercial fluorogenic substrate 4-methylumbelliferyl heptanoate (Nyfeler et al., 2003). Therefore, the choice of the substrates in the method of the invention can be adapted to optimize the sensitivity of said method.

Thus, the man skilled in the art is able to adapt the specificity and/or the sensitivity of the method of detection and/or quantification of germs responsible for axillary odour.

Depending on the level of specificity desired in the method of the invention, it is possible in said method to fit the choice of the lipase activity and the transformable substrate such as to control the detection of germs responsible for axillary odour in collected samples.

The present invention encompasses:
an *in vitro* method for the evaluation of the efficiency of a substance of interest in cosmetic formulations comprising:
   a) providing a sample obtained from a bodily surface to which a cosmetic composition comprising a substance of interest has been applied, wherein the bodily surface is skin;
   b) evaluating the presence of germs responsible for axillary odour sharing lipase activity by contacting said sample with a lipase substrate, and detecting and quantifying the amount of transformed substrate, wherein said sample is not submitted to a culture step prior to said contacting;
   c) evaluating said efficiency by comparing with results without previous application of said composition.

More preferably the lipase substrate is a C3 to C18 ester, preferably a 2-hydroxy-4-p-nitrophenoxy-butyl carboxylic acid ester, and particularly preferred it is 2-hydroxy-4-p-nitrophenoxy-butyl hexanoate or 2-hydroxy-4-p-nitrophenoxy-butyl decanoate.

Moreover, the transformed substrate is directly detectable, or it is detectable after at least one additional step following the enzymatic step.

The methods according to the present invention can be performed in a clearly reduced time span relative to the state of the art; usually maximum 3h, preferably 30 min to 2.5h are sufficient for the completion of all steps.

Said sample containing germs which are searched by the method, is derived from a bodily surface, namely skin, preferably of armpits, of an animal, preferably a human being.

The enzymatic activity shared by germs which are responsible for axillary odour is a lipase, then the substrate transformable by the enzymatic activity of the targeted germs to release a detectable product is an ester. More preferably, the ester is a C₃ to C₁₈ ester, preferably a 2-hydroxy-4-p-nitrophenoxy-butyl carboxylic acid ester, and particularly preferred it is 2-hydroxy-4-p-nitrophenoxy-butyl hexanoate or 2-hydroxy-4-p-nitrophenoxy-butyl decanoate.

The evaluation of the efficiency of a subsance of interest expected to act on said germs can be performed when said substance of interest and the sample are put together before the collection of said sample, or when said substance of interest and the sample are put together after the collection of said sample.

As axillary odor is closely related to the lipase activity of armpit germs, the efficiency of substance of interest in cosmetic formulations, e.g. deodorants or shampoos can be easily measured by the method of the invention. To this end, the presence of armpit germs sharing said lipase activity is evaluated; this is done with and without previous application of a cosmetic composition comprising a substance of interest to the site the sample suspected of containing said armpit germs is derived from.

The substances of interest can be a chemical, biological, pharmaceutical, cosmetic, veterinary or agricultural substance.

A preferred application of the above explained evaluation method, is the evaluation of the efficiency of antimicrobial substances.

In a particularly preferred method of the invention, the substances of interest to be analyzed is selected from the group consisting of anti-acne compounds, anti-acne compositions, deodorant compounds, deodorant compositions, compounds of a shampoo, or shampoo compositions, preferably of anti-dandruff shampoos.

Said method can therefore be useful to test and/or monitor the efficiency, specifically the inhibitory effect or the activator effect, of such substances of interest, in a non restrictive way, on samples collected from the surface body of an animal or a patient with a microbial infection.

That is, the method may be used to compare the efficiency of a substance of interest that is brought into contact with the targeted germs, with a negative control test, wherein the substances of interest is not applied to said germs. The effectiveness of the substances of interest can be evaluated on the basis of the difference between the effect exerted on said germs that have been, or have not been treated with the substances of interest.

For example, if a substance of interest inhibits the growth of targeted germs, the lipase activity shared by said germs generally also tends to decrease. When the lipase activity of targeted germs, which have been treated with the substances of interest is compared with the lipase activity of targeted germs that have not been treated with the substances of interest, and therefore have not experienced a growth inhibition, the determination of the efficiency of the test substance is possible.

The reduction of lipase activity can be measured easily with the described method. Thus, the method is used for confirming the presence of specific germs sharing the same lipase activity on the skin, wherein said lipase activity is indicative for the presence and quantity of germs expressing said lipase.

The use of this method allows the detection and/or quantification of targeted germs. Moreover the use of this method allows the evaluation of the efficiency of a substance of interest.

Other advantages and characteristics of the invention will emerge from reading the examples below that are part of the experimental works carried out by the applicant within the scope of the invention.

### Examples:

In the following examples, it is furthermore shown that the present invention can be used for several applications, including but not limited to:
- Detection and/or quantification of a group of organisms on skin surfaces (as shown in example 1)
- Detection and/or quantification of a group of organisms on different solid surfaces (as shown in example 2)
- Detection and/or quantification of a specific group of organisms by means of a shared esterase activity (as shown in example 3)
- Detection and/or quantification of a specific group of organisms by means of a shared protease activity (as shown in example 4)
- Detection and/or quantification of a group of *Bacillus strains* on different solid surfaces (as shown in example 5)
- Method of the evaluation of the effect of a substance of interest as a deodorant (as shown in example 6)
- Screening method of different substances of interest for their microbial decontamination efficiency (as shown in example 7)
- Kit for the culture free detection of a group of organisms sharing a common esterase/lipase activity (as shown in example 8).

### Example 1:

### Detection and/or quantification of a group of organisms on skin surfaces

The skin surface contains lipids that are used by natural skin micro-organisms as energy sources for their growth, which means that these micro-organisms contain lipases or esterases as a shared enzymatic activity.
In this experiment, lipase/esterase activity was selected as the shared activity to detect and/or quantify the naturally occurring organisms on skin. The transformable substrate chose for the detection of said activity was the 2-hydroxy-4-p-nitrophenoxy-butyl decanoate.
Microorganisms were sampled directly on the skin using a swab impregnated with aqueous acetate buffer (0.1 M, pH 5.6) containing 0.1% w/v of Triton X100. The sampling surfaces were chosen to be approximately 10 cm², on different skin surfaces such as forehead, armpit, scalp and arm. The time of sampling was approximately 1 minute.

After sampling, the swab was immersed in 200 µl of a 2-hydroxy-4-p-nitrophenoxy-butyl decanoate (Cl0-HpNPB) substrate solution (16 µl of a 20 mM stock solution in DMSO), in aqueous acetate buffer (0.1 M, pH 5.6, 184 µl). The enzymatic reaction was performed at 37°C for 1.5 hours.

After incubation, the swab was removed from the reaction mixture, and 100 µl were transferred to another vial for detection of transformed substrate: 4 µl of BSA (from an aqueous stock solution at 50 g/L), 40 µl of NaIO₄ (from an aqueous stock solution at 100 mM) and 40 µl of Na₂CO₃ (from an aqueous stock solution at 200 mM) were added and let react at room temperature for 10 minutes. The sample was centrifuged at 16,000 g for 15 minutes, the supernatant was transferred to a 96-well-microtiterplate, and the hydrolysis of the substrate was quantified by measuring the optical density at 414 nm using a Spectramax 190 microtiterplate spectrophotometer (Molecular Devices). [D. Lagarde et al., Org. Process Res. Dev., 6, pp. 441 (2002)].

### Results:

Choosing a 2-hydroxy-4-p-nitrophenoxy-butyl decanoate (C10-HpNPB) as control substrate for the quantification, it was shown that said fatty acid ester is cleaved by enzymes from group of micro organisms sampled from the armpit and other surfaces of the body using the method described above. The results of said test are described in the following table.

| | Arm | Armpit | Forehead | Scalp |
|---|---|---|---|---|
| Volunteer A | 0.116 | 0.624 | 0.321 | 0.157 |
| Volunteer B | 0.063 | 0.267 | 0.186 | 0.389 |
| Volunteer C | 0.077 | 0.185 | 0.108 | 0.082 |
| Volunteer D | 0.091 | 0.260 | 0.293 | 0.157 |
| Volunteer E | 0.057 | 0.047 | 0.164 | 0.164 |
| Control (average of 4 values): 0.049 | | | | |

| | | | | |
|---|---|---|---|---|
| Results expressed in OD at 414nm Control: Blank experiment without sampling | | | | |

Due to the usually low concentration of microorganisms on the arms, the corresponding activities are very low. In the case of samples from the armpit, forehead and scalp the average values are higher, and differences observed between the volunteers are probably due to the efficiency of their applied own personal care products.
The method of the invention can thus be used to rapidly detect and/or quantify a group of organisms that are present on skin surfaces without any culture step.

### Example 2:

### Detection and/or quantification of a group of organisms on different solid surfaces.

In this example, the method of the invention has been used to detect and/or quantify a group of organisms on different surfaces. Such application is useful to determine if a surface contains viable cultivable or non cultivable organisms, for example in microbiological quality control in food or pharmaceutical industries, or in microbiological environment monitoring (e.g. the case of a bioterrorist attack), etc.. As many micro-organisms contain esterases activity, the shared enzymatic activity selected for this example was the lipase/esterase one; and the transformable substrate for the detection of said activity was the 2-hydroxy-4-p-nitrophenoxybutyl decanoate.

Swabs were beforehand impregnated with aqueous acetate buffer 0.1M pH5.6 containing 0.1% w/v of Triton X100. Samples were collected onto several solid surfaces using two swabs hold together. One of the two swabs has been analyzed as follows: 16µL of 2-hydroxy-4-p-nitrophenoxy-butyl decanoate 20mM in DMSO have been mixed with 184µL of aqueous acetate buffer 0.1M pH5.6. The swab has been plunged in this mix and incubated for 1h30. The swab has then been removed from the reaction mixture and 100µL have been transferred to another vial. 4µL of bovine serum albumin 50mg/mL in water, 40µL NaIO₄ 100mM in water and 40µL Na₂CO₃ 200mM in water have been added. The reaction mixture has been incubated for 10 min at room temperature and centrifuged for 15 min at 16000g. The activity has then been quantified by measuring optical density at 414nm.

The results obtained with the method of the invention were then compared with a conventional microbiological assay which consist in cultivating micro-organisms that have been collected on the surface and count the amount of viable cultivable colonies (such test can obviously not estimate the amount of viable non cultivable organisms or organisms that are difficult to cultivate).

For this comparison, the second swab has been incubated in 200µL of LB medium for 1h at 37°C in order to release micro-organisms from the swab. The liquid has then been spread on Petri dishes containing LB medium. The dishes have been incubated at 37°C and the resulting colonies have been counted after 24h of incubation.

Results are shown in the following table and in Figure 1:

| analyzed surface | activity | colony count |
|---|---|---|
| handle of freezer's door | 0.069 | 75 |
| handle of micro-pipette | 0.052 | 13 |
| bench tab | 0.05 | 0 |
| glasses | 0.048 | 0 |
| window of laminar flow hood | 0.049 | 0 |
| computer keyboard | 0.079 | 74 |
| forehead person n°1 | 0.212 | 296 |
| forehead person n°2 | 0.236 | 281 |

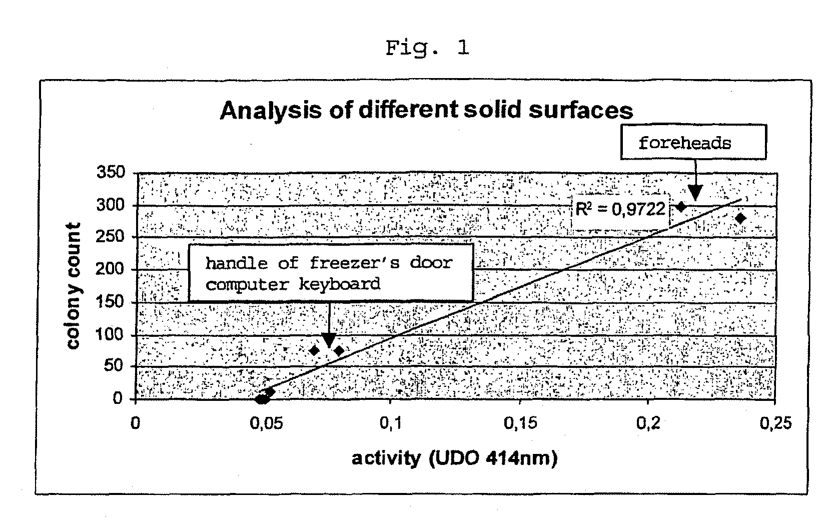

These results show that the method allows the detection and/or quantification of unidentified viable cultivable or non cultivable organisms on several kinds of surfaces (without any culture step) including surfaces which are known to be not heavily contaminated. The same level of activity (cultivable and non cultivable organisms) has been detected with the present method for handle of micro-pipette, glasses and window of laminar flow hood, while conventional microbiological assay detected 13 cultivable organisms on handle of micro-pipette but no cultivable organisms on glasses and window of laminar flow hood.

The method allows thus to monitor the amount of a group of viable cultivable and non cultivable organisms on a surface by sampling the surface at different times.

### Example 3:

### Detection and/or quantification of a specific group of organisms by means of a shared esterase activity

In this example, the detection of two *B. subtilis* strains by means of their shared esterase activity was achieved in comparison of the detection of an *E. coli* strain. As the two *B. subtilis* strains share a common esterase activity, but not the *E. coli* strain, this application aims to demonstrate the feasibility of the detection of a specific group of organisms *(B. subtilis)* versus another one *(E. coli)* with the present method.
For this demonstration of the detection of specific groups of organisms by means of the method, cell preparations of three bacterial strains were realized by one overnight culture:
- *Bacillus subtilis* strain ATCC 12711 in DSM Medium 630: modified Thermus 162 medium at 30°C
- *Bacillus subtilis globigii* in Bacto Nutrient Broth at 30 °C
- *Escherichia coli* strain MC1061 in LB medium at 37 °C.

Correlation of cell counts with OD600 was previously established for these strains by measuring the OD600 and spreading serial dilutions of these strains on Petri dishes and counting colonies. These cell preparations were then quantified by measurement of OD600 of appropriate dilutions of these cells.

1mL of each cell preparation has been centrifuged during 5 min at 13000g. The pellet has been resuspended in 10µL of culture medium. 8µL of 2-hydroxy-4-p-nitrophenoxy-butyl hexanoate 20mM in DMSO and 74µL of PBS have been added. Negative controls for each strains are 10µL of culture medium. Reaction mixtures have been incubated for 40min at 30°C *(Bacillus samples)* or 37°C *(Escherichia samples).* 4µL of bovine serum albumin 50mg/mL in water, 40µL NaIO₄ 100mM in water and 40µL Na₂CO₃ 200mM in water have been added. The reaction mixture has been incubated for 10 min at room temperature and centrifuged for 15 min at 16000g. The activity has then been quantified by measuring optical density at 414nm.

Results are shown in the following table.

| Strain | activity (OD₄₁₄/10⁷ cfu) |
|---|---|
| *B. subtilis globigii* | 0.0600 |
| *B. subtilis* | 0.0779 |
| *E. coli* | 0.0002 |

These results show that an appropriate choice of the enzymatic activity allows the specific detection of a group of micro-organisms sharing this activity (here *Bacillus strains*) versus other strains (here *E. coli*) that do not share this activity.

### Example 4:

### Detection and/or quantification of a specific group of organisms by means of a shared protease activity

In this example, the detection of two *Bacillus strains* by means of their shared protease activity was achieved in comparison of the detection of an E. coli strain. As the two *Bacillus strains* share a common protease activity, but not the *E. coli strain,* this application aims to demonstrate the feasibility of the detection of a specific group of organisms *(Bacillus)* versus another one (*E. coli*) with the present method.
For this demonstration of the detection of specific groups of organisms by means of the method, cell preparations of three bacterial strains were realized by one overnight culture:
- *Bacillus subtilis* strain ATCC 12711 in DSM Medium 630: modified Thermus 162 medium at 30 °C
- *Bacillus subtilis globigii* in Bacto Nutrient Broth medium at 30 °C
- *Escherichia coli* strain MC1061 in LB medium at 37 °C.

Correlation of cell counts with OD600 was previously established for these strains by measuring the OD600 and spreading serial dilutions of cells preparations of these strains on Petri dishes and counting colonies. The amounts of cells have then been quantified by measurement of OD600 of appropriate dilutions of these cell preparations.

These cells have been diluted in culture medium in order to obtain suspensions containing 107, 106, 105cfu/200µL. 200µL of these suspensions have been spread in empty plastic Petri dishes and allowed to dry completely. Negative controls are empty plastic Petri dishes spread with 200µL of culture medium.

Samples have been collected onto the Petri dishes using swabs beforehand impregnated with PBS containing 0.1% w/v of Triton X100. Protease activity has been measured using the following protocol: 100 µL of 0.2M Tris HCl pH 7.8, 20mM CaCl₂ and 100 µL of 0.04% w/v casein resorufin, as the substrate, have been mixed. The swabs have been immersed in this mix and incubated at 37°C for *E. coli* samples and at 30°C for *Bacillus* samples for 2h. 20µL of the reaction mixture have been mixed with 48µL of trichloroacetic acid 5% w/v in water, incubated at 37°C for 10min and centrifuged at 3500 rpm for 10min. 40µL of the supernatant have been mixed with 60µL of 0.5M Tris HCl pH8.8. The activity has been quantified by measuring the fluorescence of the samples (excitation wavelength 565nm, emission wavelength 594nm).

Results are shown in Figure 2.

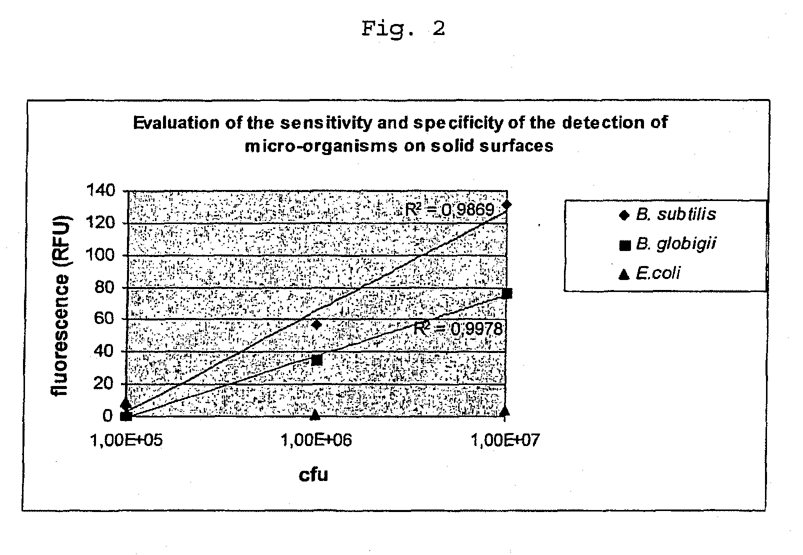

These results show that an appropriate choice of the enzymatic activity allows the specific detection of a group of micro-organisms sharing this activity (here *Bacillus strains*) versus other strains (here *E. coli*) that do not share this activity. In addition, the detection protocol including collection of the sample on plastic surfaces is shown here to be quantitative.

### Example 5:

### Detection and/or quantification of a group of Bacillus strains on different solid surfaces.

As in example 3, the detection of two *Bacillus strains* by means of their shared esterase activity was achieved either on plastic and glass surfaces, to check if the chemical nature of the surface could have on impact on this detection method.
The cell preparations of the two strains were realized by one overnight culture:
- *Bacillus subtilis* strain ATCC 12711 in DSM Medium 630: modified Thermus 162 medium at 30°C
- *Bacillus subtilis globigii* in Bacto Nutrient Broth medium at 30 °C.

Correlation of cell counts with OD600 was previously established for these strains by measuring the OD600 and spreading serial dilutions of cultures of these strains on Petri dishes and counting colonies. These cell preparations have then been quantified by measurement of OD600 of appropriate dilutions of these cells.

These cells have been diluted in PBS in order to obtain suspensions containing 0,5.107cfu / 200µL. 200µL of these suspensions have been spread on solid plastic surfaces and on solid glass surface and allowed to dry completely. Negative controls are solid plastic surfaces spread with 200µL of PBS.

Samples have been collected onto the surfaces using swabs beforehand impregnated with PBS containing 0.1% w/v of Triton X100. 16µL of 2-hydroxy-4-p-nitrophenoxy-butyl hexanoate 20mM in DMSO have been mixed with 184µL of aqueous PBS buffer 0.1M pH7.3. The swab has been plunged in this mix and incubated for 1h30. The swab has then been removed from the reaction mixture and 100µL have been transferred to another vial. 4µL of bovine serum albumin 50mg/mL in water, 40µL NaIO₄ 100mM in water and 40 µL Na₂CO₃ 200mM in water have been added. The reaction mixture has been incubated for 10 min at room temperature and centrifuged for 15 min at 16000g. The activity has then been quantified by measuring optical density at 414nm. Results are shown in the following table.

Results are shown in the following table.

| | glass | plastic |
|---|---|---|
| *B. subtilis globigii* | 0.062 | 0.038 |
| *B. subtilis* | 0.069 | 0.029 |

These results show that the shared enzymatic activity of a group of organisms can be detected on any kind of surface.

### Example 6:

### Method of the evaluation of the effect of a substance of interest as a deodorant

Naturally occurring micro-organisms on skin, and more specifically on armpits, are known to generate odours. Several substances (incorporated in deodorants) can be used to slow down the growth of this group of organisms in order to lower the odour formation. The conventional methods to evaluate the efficiency of new substances as active deodorants are either a sniff test, which consists in smelling treated or untreated armpit with said substances, or an in vitro microbiological assay.
In this experiment, polyglyceryl-3 caprylate (Tego Cosmo P813, Goldschmidt) was assayed on armpits to evaluate its modulatory effect on the growth of a group of skin micro organisms, as a new active deodorant. The method of the invention as been used to evaluate the efficiency of this substance of interest.
As described in example 1, the selected shared enzymatic activity was the lipase/esterase; and the transformable substrate for the detection of said activity was the 2-hydroxy-4-p-nitrophenoxy-butyl decanoate.

The deodorant was applied to one armpit of a test person (2% Laureth-23 (TEGO Alkanol L 23P, Goldschmidt), 0.5% polyglyceryl-3 caprylate, 97.5% water). The other armpit was treated with a control (2% Laureth-23, 98% water) and was used as a reference. In the evening the test persons had a shower. Then deodorant and control were applied. In the morning deodorant and control were used again. At noon the reduction of group of microorganisms count relative to the reference was measured using the method and kit described above.

Results are shown in the following table.

| | Deodorant | Control | Control - Deodorant |
|---|---|---|---|
| Person A | 0.51 | 1.04 | 0.53 |
| Person A | 0.11 | 0.29 | 0.18 |
| Person A | 0.27 | 1.05 | 0.78 |
| Person B | 0.67 | 0.72 | 0.05 |
| Person B | 0.12 | 0.18 | 0.06 |
| Person B | 0.13 | 0.75 | 0.62 |
| Person C | 0.51 | 1.04 | 0.53 |
| Person C | 0.48 | 1.11 | 0.63 |
| Person C | 0.45 | 1.10 | 0.65 |

In 7 of 9 cases a significant reduction of lipase activity was found.

The results obtained with the method of the invention were then compared to a conventional sniff test and an antimicrobial *in vitro test.*

### Comparison with the Sniff test:

20 test persons washed their axilla. The odor was evaluated by three experts. Then a polyglyceryl-3 caprylate solution (2% Laureth-23 (TEGO Alkanol L 23P, Goldschmidt), 0.3% polyglyceryl-3 caprylate, 97.7% water) was applied to one armpit of each test person. The other armpit was left untreated and was used as a reference. The axilla odor was evaluated after 6 and 24h. A significant improvement of axillary odor was found compared to the untreated axilla.

The results correspond to the results gained using the method and kit described above. The results obtained with the method of the invention are reproducible, quantitative and objective which is not necessary by the case of the sniff test that even with qualified testers, hardly remains quantitative due to its subjective nature. Moreover the method of the invention is easier and more pleasant to perform.

### Comparison with the in vitro microbiological method:

An EuAB test for antimicrobial activity was performed with *Corynebacterium xerosis* (DSM 20743), *Staphylococcus epidermidis* (DSM 3269) and *Candida albicans* (ATCC 10231). As culture medium CSL (Casein peptone-soybean meal peptone solution), CSA (Casein peptone-soybean meal pepton agar) and Sabouraud-glucose broth/agar was used.

As dilution liquid NaCl-peptone buffer solution with inactivator (3% Tween 80, 0.3% lecithin, 0.1% histidine and 0.5% Na thiosulfate) was used. Polyglyceryl-3 caprylate was tested in 0.3% (in CSL) concentration. The test solution was prepared one day before investigation. For this, 100 ml of CSL was heated to 60°C in a water bath. 0.3 g was weighed into 100 ml of CSL at 60°C. The preparations were shaken vigorously by hand and left overnight at 30°C in an incubator. *Corynebacterium xerosis* was cultivated over 3 to 4 days. Other microbes were isolated in broth or by elutriation. For each test microbe, 20 ml of each test solution were introduced into sterile 50 ml brown glass bottles with glass beads and contaminated with 0.2 ml of microbe suspension. As controls, 20 ml of CSL were carried over per test microbe without sample.

The contaminated samples were shaken for 3 min on a shaking machine and kept in an incubator at 30°C until removed. At the removal points (1, 2, 3, 24 and 48 hours) 1 ml was taken from each preparation and transferred in each case to 9 ml of NaCl-peptone buffer solution with inactivator and the colony number was determined. The 0 hour values given were the colony numbers of the test microbe suspension used taking into consideration the 10⁻² dilution upon sample contamination.

The results are given in enclosed Figure 3.

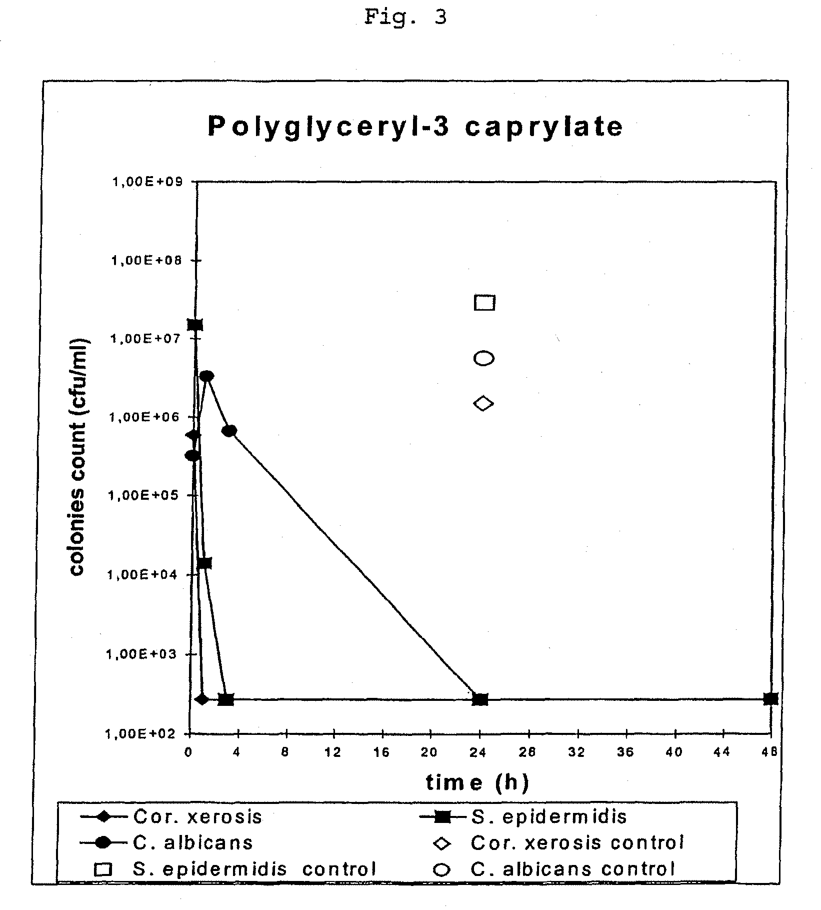

Also shown is the microbe population of an active-ingredient-free blind sample as control value after incubation for 24 hours.
A reduction of all bacteria count from 10⁷ to less than 270 could be found after 24h.

This *in vitro* microbiological method also proves the antimicrobial efficiency of polyglyceryl-3 caprylate.

Therefore all three methods show concurrent results. But the test method described in this invention is by far the fastest and easiest of all three, and clearly demonstrates the effect of the substance of interest.

### Example 7:

### Screening method of different substances of interest for their microbial decontamination efficiency.

The method has been applied to evaluate the microbial decontamination efficiency of different cleaning substances. For this purpose, a benchmark bacterial strain having an esterase activity (isolated by traditional microbiological methods from samples collected from human foreheads) has been used to artificially contaminated surfaces. This strain has been prepared by an overnight incubation at 37°C in LB medium. 200µL of this cell preparation, corresponding to 6.10⁷ cfu have been spread on five plastic surfaces and allowed to dry completely. After drying, one of the plastic surface has been cleaned with a wipe impregnated with water, a second one has been cleaned with a wipe impregnated with liquid soap, a third one has been cleaned with a wipe impregnated with 70% v/v ethanol in water, a fourth one has been cleaned with a wipe impregnated with bleach and the last one has not been cleaned at all.

Samples have then been collected onto the different plastic surfaces using swabs beforehand impregnated with PBS containing 0.1% w/v of Triton X100. 16µL of the transformable substrate (2-hydroxy-4-p-nitrophenoxy-butyl hexanoate 20mM in DMSO) have been mixed with 184µL of aqueous PBS buffer 0.1M pH7.3. The swab has been plunged in this mix and incubated for 1h at 37°C. The swab has then been removed from the reaction mixture and 100µL have been transferred to another vial. 4µL of bovine serum albumin 50mg/mL in water, 40µL NaIO₄ 100mM in water and 40µL Na₂CO₃ 200mM in water have been added. The reaction mixture has been incubated for 10 min at room temperature and centrifuged for 15 min at 16000g. The activity has then been quantified by measuring optical density at 414nm.

Results are shown in the following table.

| | |
|---|---|
| cleaning | activity |
| none | 3.474 |
| water | 0.100 |
| soap | 0.121 |
| 70% ethanol | 0.195 |
| bleach | 0 |

These results show that the method is suitable to screen for different substances suspected to act on organisms, and to select the best substance (in that case the bleach) on the basis of the amount of transformed substrate.

### Example 8:

### Kit for the culture free detection of a group of organisms sharing a common esterase/lipase activity.

Such a kit contains swabs that are soaked in 135 □L of a sampling buffer (which can be for example PBS containing 0.1% w/v of Triton X100). A swab is used to collect on a surface the potential group of organisms, and is then dropped in an incubation tube containing 16µL of 2-hydroxy-4-p-nitrophenoxy-butyl hexanoate 20mM in DMSO have been mixed with 184µL of aqueous PBS buffer 0.1M pH7.3 (dilution 1/10^{e}). The incubation tube is closed and incubated at the 37°C 90 minutes to enable the transformation of the substrate by the shared activity.

After incubation, the swab is removed, and the liquid is transferred in a revelation tube containing 4µL of bovine serum albumin 50mg/mL in water, 40µL NaIO₄ 100mM in water and 40µL Na₂CO₃ 200mM in water(another way to reveal the shared activity is to directly drop the revelation solution into the incubation tube).
The activity is then observed by visual analysis of the yellow color as compared to a reference test run with a sterile swab.

Such a kit can be used to detect esterases or lipases containing micro-organisms on any kind of surfaces, including skin.

## Claims

1. An *in vitro* method for the evaluation of the efficiency of a substance of interest in cosmetic formulations comprising:
a) providing a sample obtained from a bodily surface to which a cosmetic composition comprising a substance of interest has been applied, wherein the bodily surface is skin;
b) evaluating the presence of germs responsible for axillary odour sharing lipase activity by contacting said sample with a lipase substrate, and detecting and quantifying the amount of transformed substrate, wherein said sample is not submitted to a culture step prior to said contacting;
c) evaluating said efficiency by comparing with results without previous application of said composition.

2. The method according to claim 1, wherein the lipase substrate is a C3 bis C18 ester.

3. The method according to claim 2, wherein the lipase substrate is the 2-hydroxy-4-p-nitrophenoxy-butyl decanoate.

## Patentansprüche

1. *In-vitro*-Verfahren für die Auswertung der Wirksamkeit einer interessierenden Substanz in Kosmetikformulierungen, umfassend die folgenden Schritte:
a) Bereitstellen einer Probe von einer Körperoberfläche, auf die eine Kosmetikzusammensetzung enthaltend eine interessierende Substanz aufgetragen worden ist, wobei es sich bei der Körperoberfläche um die Haut handelt;
b) Auswerten des Vorhandenseins von Mikroorganismen, die für für Achselgeruch mitverantwortliche Lipaseaktivität verantwortlich sind, durch In-Kontakt-Bringen der Probe mit einem Lipasesubstrat, und Nachweisen und quantitatives Bestimmen der Menge an umgewandeltem Substrat, wobei mit der Probe vor dem In-Kontakt-Bringen kein Kultivierungsschritt durchgeführt wurde;
c) Auswerten der Wirksamkeit durch Vergleichen mit Ergebnissen ohne vorheriges Auftragen der Zusammensetzung.

2. Verfahren nach Anspruch 1, wobei es sich bei dem Lipasesubstrat um einen C3- bis C18-Ester handelt.

3. Verfahren nach Anspruch 2, wobei es sich bei dem Lipasesubstrat um 2-Hydroxy-4-p-nitrophenoxybutyl-decanoat handelt.

## Revendications

1. Méthode d'évaluation *in vitro* de l'efficacité d'une substance d'intérêt dans des formulations cosmétiques, comprenant les étapes consistant à :
a) se procurer un échantillon obtenu d'une surface corporelle sur laquelle une composition cosmétique comprenant une substance d'intérêt a été appliquée, la surface corporelle étant la peau ;
b) évaluer la présence de germes responsables de l'odeur axillaire ayant en commun une activité lipase, en mettant en contact ledit échantillon avec un substrat de lipase et en détectant et quantifiant la quantité de substrat transformé, ledit échantillon n'étant pas soumis à une étape de mise en culture avant ladite mise en contact ;
c) évaluer ladite efficacité en réalisant une comparaison avec des résultats obtenus sans application préalable de ladite composition.

2. Méthode selon la revendication 1, le substrat de lipase étant un ester en C3 à C18.

3. Méthode selon la revendication 2, le substrat de lipase étant le décanoate de 2-hydroxy-4-p-nitrophénoxy-butyle.
